# EUROPÄISCHE PATENTANMELDUNG

(11) **EP 0 586 833 A1**
(43) Veröffentlichungstag der Anmeldung: **16.03.1994**
(21) Anmeldenummer: 93111262.7
(22) Anmeldetag: 14.07.1993
(51) Int. Cl.: A61M 5/32

(54) **Einrichtung zum Entsorgen von Kanülen**

(30) Priorität: 11.09.1992 DE 9212259 U
(71) Anmelder: KIRCHNER & WILHELM GMBH & CO., D-71679 Asperg (DE)
(72) Erfinder: Kirchner, Georg, D-78628 Rottweil (DE)
(74) Vertreter: Schmid, Berthold, Dipl.-Ing.

(57) **Zusammenfassung**

Um eine ungefährliche Entsorgung gebrauchter Injektions-Kanülen (13) zu erreichen und eine Kontamination der Umwelt zu verhindern, ist eine Einrichtung zum Entsorgen der Kanüle (13) einer bereits gebrauchten Injektionsspritze gekennzeichnet durch eine der Aufnahme der gebrauchten Kanüle (13) dienende Box (1) oder dgl., die eine mindestens teilweise aus einem elastischen Material bestehende und damit von der Kanüle (13) durchstechbare Wandung (4, 5, 8) aufweist.

## Beschreibung

Nach der Verwendung von Injektionsspritzen ergeben sich für deren Benutzer zwei Probleme. Einerseits soll die gebrauchte Injektions-Kanüle nicht noch einmal verwendet werden, was im allgemeinen durch ein Abknicken der bereits gebrauchten Injektions-Kanüle verhindert wird. Andererseits aber muß auch noch vermieden werden, daß eine Kontamination der gebrauchten Kanüle mit der Umwelt möglich ist. Dieses Problem ist besonders groß bei der Entsorgung der festmontierten Kanülen von in großen Mengen verwendeten Einmalgehrauchspritzen, wie diese insbesondere auch von Diabetikern benutzt werden. Hierbei bilden auch die abgeknickten Kanülen eine besondere Gefahrenquelle für Verletzungen, da die aus Kunststoff bestehenden Entsorgungssäcke leicht von den benutzten Kanülen durchstochen werden.

Aufgabe der vorliegenden Erfindung ist es somit, diesen bisher noch bestehenden Übelstand zu beseitigen und eine Einrichtung zu schaffen, mittels welcher die gebrauchten Injektions-Kanülen völlig ungefährlich entsorgt werden können und keine Kontamination mit der Umwelt mehr zu befürchten ist.

Diese Aufgabe wird erfindungsgemaß gelöst durch eine der Aufnahme der gebrauchten Kanülen dienende Box oder dgl., welche eine mindestens teilweise aus einem elastischen Material bestehende Wandung aufweist, durch die jede bereits gebrauchte Kanüle so weit in die Box hineingestochen werden kann, bis auch das der Injektionsspritze zugekehrte Ende der Kanüle fest von dem elastischen Material umgeben und festgehalten wird. Anschließend kann durch ein Hin- und Herbewegen der außerhalb der Box befindlichen Injektionsspritze die Kanüle abgebrochen werden, welche auch weiterhin geschützt in der Box verbleibt und durch das elastische Material festgehalten wird. Sowohl die Einmalgebrauchsspritze als auch die mit nur einer oder auch mehreren gebrauchten Kanülen gefüllte Box können dann unbesorgt dem Müll zugeführt werden.

Bei einer besonders interessanten Gestaltung dieser Box weist dieselbe eine das Einführen der Kanüle in das Boxinnere ermöglichende Öffnung auf, die durch mindestens eine aus dem elastischen Material bestehende Platte oder dgl. abgedeckt ist.

Gegebenenfalls ist es aber auch möglich, diese Öffnung durch zwei in einem Abstand voneinander angeordnete Platten abzudecken, wobei sich dann zwischen diesen beiden Platten zweckmäßig zusätzlich eine aus dem gleichen oder einem anderen elastischen Material bestehende, die eingeführte Kanüle festhaltende Einlage befindet, welche beispielsweise durch eine in der Box befindliche seitliche Öffnung in die zwischen diesen beiden Platten befindliche Kammer eingeschoben werden kann.

Um auch beim Hineinstechen der gebrauchten Kanüle in das elastische Material der Entsorgungsbox eventuelle Verletzungen des Benutzers zu vermeiden, ist es ferner noch von Vorteil, wenn die durch das elastische Material abgedeckte Öffnung der Box von einem besonderen Fingerschutz umgeben ist.

Statt durch die oben erwähnten, aus dem elastischen Material bestehenden Platten kann die Öffnung der Entsorgungsbox aber auch durch einen aus einem gleichartigen Material bestehenden Stopfen oder dgl. verschlossen sein, wobei dieser Stopfen dann zweckdienlich soweit in die Box hineingeschoben ist, daß deren Rand über die äußere Stirnfläche des Stopfens hinausragt und so bereits den zuvor schon erwähnten Fingerschutz bildet.

Ist die Entsorgungsbox als beiderseits offene Hülse ausgebildet, deren eine Öffnung durch den zuvor schon erwähnten Stopfen verschlossen ist, so kann diese Hülse mit ihrem anderen freien Ende derart auf den Ansatz der Injektionskanüle aufgesteckt werden, daß diese vollständig abgedeckt ist. Eine solche Ausbildung und Verwendung dieser hülsenförmigen Entsorgungsbox ist dabei insbesondere für Einmalgebrauchsspritzen geeignet, da die Entsorgungsbox dann schon beim Versand und vor dem Gebrauch der Injetionsspitze als die Injektionskanüle abdeckende Schutzhülse dient. Um eine Berührung der abgebrochenen Kanüle mit völliger Sicherheit auszuschließen, ist nach einem weiteren Merkmal der Erfindung die Box an der Einstichseite mit einem Deckel verschließbar.

Weitere Einzelheiten der vorliegenden Erfindung ergeben sich aus der folgenden Beschreibung. Es zeigen:
- Fig. 1: die Seitenansicht einer ersten Ausführungsform der Entsorgungsbox,
- Fig. 2: einen Schnitt gemäß der Linie II-II,
- Fig. 3: einen Schnitt der Entsorgungsbox gemäß der Linie III-III;
- Fig. 4: den Schnitt einer Einmalgebrauchsspritze mit der als Schutzhülle dienende Entsorgungsbox
und
- Fig. 5: das Einführen der gebrauchten Injektionskanüle in die Entsorgungsbox.

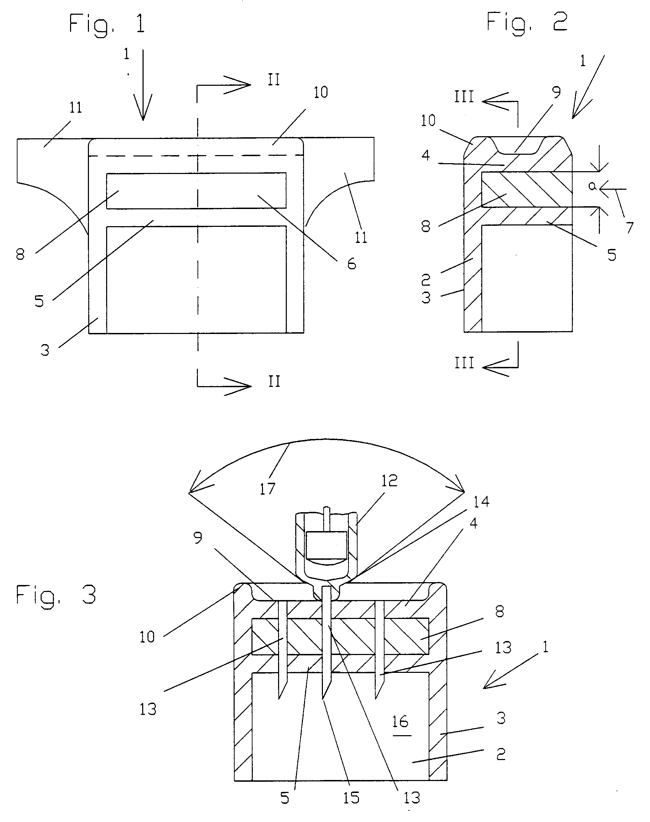

Die in den Figuren 1 bis 3 dargestellte Entsorgungsbox 1 ist kastenförmig ausgestaltet und weist eine Rückwand 2 sowie die beiden Seitenwände 3 auf. Oben ist diese Entsorgungsbox 1 durch zwei in einem Abstand a voneinander angeordnete Platten 4 und 5 abgedeckt, zwischen welche durch die Seitenöffnung 6 in Richtung des Pfeiles 7 eine aus einem elastischen Kunststoff bestehende Einlage 8 eingeschoben ist. An der Oberseite 9 der oberen Platte 4 ist dabei ein in sich geschlossener, als Fingerschutz dienender Rand 10 angeformt. Um sowohl einen sicheren Halt der Entsorgungsbox 1 in der Hand des Benutzers zu gewährleisten als auch die Finger des Benutzers zu schützen, sind an den beiden Seitenwänden 3 der Entsorgungsbox 1 noch zwei nach außen hin vorstehende Vorsprünge 11 angeformt.

Ist mittels der in der Fig. 3 teilweise gezeigten Einmalgebrauchsspritze 12 die vorgesehene Injektion durchgeführt worden, so wird die Kanüle 13 dieser Infusionsspritze 12 in der in Fig. 3 dargestellten Weise soweit von oben her durch die Platten 4 und 5 sowie die zwischen diesen befindliche elastische Einlage 8 hindurchgestochen, bis die Spritze 12 mit ihrem Hals 14 an der Oberseite 9 der oberen Platte 4 anliegt und die Kanülenspitze 15 in das Innere 16 der Entsorgungsbox 1 hineinragt. Wird anschließend die Infusionsspritze 12 in Richtung des Pfeiles 17 hin und her geschwenkt, so bricht die Kanüle 13 ab und die Infusionsspritze 12 kann von der Entsorgungsbox 1 abgehoben werden. Die Kanüle 13 dagegen verbleibt in dem durch die beiden Platten 4 und 5 sowie der Einlage 8 gebildeten Boxoberteil, wobei die Kanülenspitze 15 sicher von den beiden Wandungen 2 und 3 der Entsorgungsbox 1 abgedeckt wird.

Infolge der kastenähnlichen Gestaltung dieser Entsorgungsbox 1 ist es dabei ohne weiteres möglich, daß in diese noch weitere Injektionskanülen 13 hineingesteckt werden.

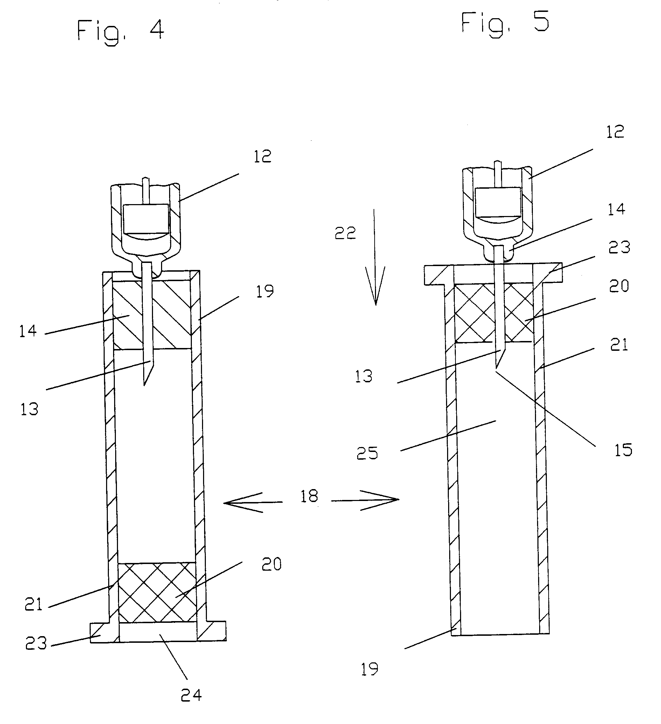

Die Fig. 4 zeigt eine gleichartige Injektionsspritze 12 vor dem Aufziehen der vorgesehen Injektionsflüssigkeit. In diesem Fall ist die Injektionskanüle 13 durch eine hülsenartige Entsorgungsbox 18 abgedeckt, wobei diese Entsorgungsbox 18 mit ihrem offenen Ende 19 auf den Hals 14 der Injektionsspritz 12 aufgesteckt ist und so die Injektionskanüle 13 auch schon bei deren Transport sicher abdeckt.

Ist die vorgesehene Infusion mittels der Injektionsspritze 12 durchgeführt worden, so wird diese Entsorgungsbox 18 umgedreht, so daß nunmehr das andere durch einen aus einem elastischen Material bestehenden Stopfen 20 verschlossene Ende 21 nach oben gerichtet ist. Nunmehr kann wie bei der Entsorgungsbox 1 die Injektionskanüle 13 von oben her in Richtung des Pfeiles 22 in die von einem Rand 23 umgebene Öffnung 24 der Entsorgungsbox 18 eingeführt und durch den Stopfen 20 hindurchgestochen werden. Anschließend wird die Injektionsspritze 12 in der bereits im Zusammenhang mit der Fig. 3 beschriebenen Weise wieder hin und her geschwenkt, so daß die Injektionskanüle 13 abbricht und auch nach dem Abheben der Injektionsspritze 12 in der Entsorgungsbox 18 verbleibt. Auch in diesem Fall ist die in das Innere 25 der Entsorgungsbox 18 hineinragende Spitze 15 der gebrauchten Infussionskanüle 13 vollständig von der Entsorgungsbox 18 umgeben und gegen jede eventuelle Berührung mit der Umwelt geschützt.

## Patentansprüche

1. Einrichtung zum Entsorgen der Kanüle einer bereits gebrauchten Injektionsspritze, gekennzeichnet durch eine der Aufnahme der gebrauchten Kanüle (13) dienende Box (1, 18) oder dgl., die eine mindestens teilweise aus einem elastischen Material bestehende und damit von der Kanüle (13) durchstechbare Wandung (4, 5, 8) aufweist.

2. Einrichtung nach Anspruch 1, dadurch gekennzeichnet, daß die Box (1, 18) hülsen- oder kastenartig ausgebildet ist.

3. Einrichtung nach Anspruch 1 und/oder 2, dadurch gekennzeichnet, daß die Box (1) eine das Einführen der Kanüle (13) in das Boxinnere (16) ermöglichende öffnung aufweist, die durch mindestens eine aus dem elastischen Material bestehende Platte (4, 5) oder dgl. abgedeckt ist.

4. Einrichtung nach Anspruch 3, dadurch gekennzeichnet, daß die öffnung (6) durch zwei in einem Abstand (a) von einander angeordnete Platten (4, 5) oder dgl. abgedeckt ist.

5. Einrichtung nach Anspruch 4, dadurch gekennzeichnet, daß sich zwischen den beiden Platten (4, 5) eine zusätzliche, aus dem gleichen oder auch einem anderen elastischen Material bestehende. die eingeführte Kanüle festhaltende Einlage (8) befindet.

6. Einrichtung nach Anspruch 5, dadurch gekennzeichnet, daß zwischen den beiden Platten (4, 5) in der benachbarten Wandung (3) der Box (1) eine Seitenöffnung (6) zum Einführen Einlage (8) vorgesehen ist.

7. Einrichtung nach einem oder mehreren der Ansprüche 1 bis 6, dadurch gekennzeichnet, daß die das Einführen der Kanüle (13) in die Box (1, 18) ermöglichende öffnung (9, 24) von einem Fingerschutz (10, 11, 23) umgeben ist.

8. Einrichtung nach Anspruch 7, dadurch gekennzeichnet, daß der Fingerschutz aus die rechteckige öffnung (9) der kastenförmigen Box (1) vollständig oder auch nur teilweise umgebenden, über diese öffnung (9) vorstehenden Leisten (10) oder dgl. besteht.

9. Einrichtung nach Anspruch 7, dadurch gekennzeichnet, daß der Fingerschutz aus einer die runde öffnung (24) der hülsenartigen Box (18) umgebenden, über diese öffnung (24) vorstehenden Rand (23) besteht.

10. Einrichtung nach den Ansprüchen 8 und/oder 9, dadurch gekennzeichnet, daß mehrere Leisten bzw. mehrere Ränder (10, 11, 23) vorgesehen sind und diese einerseits entgegen der Einstichrichtung (22) und andererseits seitlich über die Wandung (3) der Box (1, 18) vorstehen.

11. Einrichtung nach einem oder mehreren der Ansprüche 1 bis 3, dadurch gekennzeichnet, daß die öffnung der Box durch einen aus einem elastischen Material und damit von der Kanüle durchstechbaren Stopfen oder dgl. verschlossen ist.

12. Einrichtung nach Anspruch 11, dadurch gekennzeichnet, daß die Box als beiderseits offene Hülse ausgebildet ist, deren eine öffnung durch den aus dem elastischen Material bestehenden Stopfen verschlossen und deren dieser öffnung abgekehrtes freies Ende auf den Ansatz einer Injektionsspritze aufsteckbar ist.

13. Einrichtung nach einem oder mehreren der vorhergehenden Ansprüche, dadurch gekennzeichnet, daß die Box an der Einstichseite mit einem Deckel verschließbar ist.

14. Einrichtung nach einem oder mehreren der vorhergehenden Ansprüche, dadurch gekennzeichnet, daß die Box an beiden Enden mit elastischem Material verschlossen ist und beidseitig verwendet werden kann.
